# EUROPEAN PATENT SPECIFICATION

(11) **EP 0 384 769 B1**
(45) Date of publication and mention of the grant of the patent: **24.04.1996**
(21) Application number: 90301949.5
(22) Date of filing: 23.02.1990
(51) Int. Cl.: C07D 207/46, C07D 401/12, C07D 401/04, C07D 417/12, G01N 33/534, A61K 51/00

(54) **Protein labelling**
Markierung von Proteinen
Marquage de protéines

(30) Priority: 24.02.1989 US 315270
(43) Date of publication of application: 29.08.1990
(73) Proprietor: JOHNSON MATTHEY PUBLIC LIMITED COMPANY, London, EC1N 8JP (GB)
(72) Inventor: Schwartz, David A., Exton, Pennsylvania 19341 (US); Abrams, Michael J., Glenmore, Pennsylvania 19343 (US); Giandomenico, Christen M., Exton, Pennsylvania 19341 (US); Zubieta, Jan A., Albany, New York 12202 (US)
(74) Representative: Wishart, Ian Carmichael

(56) References cited:
- EP-A- 0 247 866
- EP-A- 0 271 806
- DE-A- 3 216 026

## Description

### Background of the Invention

This invention relates to bifunctional compounds capable of linking metal ions, particularly technetium and rhenium, to biologically useful molecules.

Because of their high biological specificity, certain macromolecules (e.g., monoclonal antibodies) have been used to target radioisotopes to specific in vivo sites for the purpose of imaging and/or therapy. The use of the metastable isotope of technetium, ^{99m}Tc, in diagnostic nuclear medicine is well established and the beta-emitting isotopes of rhenium ¹⁸⁶Re, ¹⁸⁸Re and ¹⁸⁹Re can be used therapeutically. A number of methods for attaching technetium to macromolecules have been described. Some of these methods involve the reduction of disulfide groups in the macromolecule (usually an immunoglobulin) to thiols and the subsequent use of these groups to bind reduced Tc (e.g., McKenzie et al., International Publication #WO 87/04164; and Bremer et al., EPO 271 806 A2). Methods of this type have several potential disadvantages. The reduction of disulfide units can lead to protein denaturation and a subsequent loss in biological specificity. Also, the method cannot be used to label macromolecules lacking disulfide moieties.

Alternatively, ^{99m}Tc can be linked to macromolecules via bifunctional chelates such as DTPA (D. Lanteigne and D.J. Hnatowich, Int. J. Appl. Radiat. Isot., Vol. 35(7) p.617, (1984), chelating thiosemicarbazones (Y. Arano et al., Int. J. Nucl. Med. Biol., Vol. 12 p.425, (1985), and diamidedithiol ligands (A. Fritzberg, European Patent Appl. EP 188256 2A). Problems associated with these methods include significant nonspecific binding of technetium (binding to the protein at sites other than the chelating group) and slow kinetics of Tc-labelling.

EP-A-247866 concerns radioisotope- (such as Tc or Re) labelled antibody fragments for diagnostic and/or therapeutic uses. A bi-functional chelating coupling agent is reacted with the antibody or fragment to form an antibody conjugate, which is complexed with the radioisotope.

EP-A-271806A teaches that Tc-99m can be used to label organ-specific substances such as antibodies or antibody fragments, providing that such substance is pretreated with or coupled to a complexing agent for Tc-99m.

Accordingly, it is the object of the present invention to provide new bifunctional molecules having hydrazine or hydrazide groups and protein reactive groups which can be used to link metal ions, such as ^{99m}Tc, to macromolecules.

Another object of the present invention is to provide a method for labelling macromolecules with metal ions in which binding of the metal at sites other than the chelating group is minimal, and in which labelling occurs at a relatively fast rate (less than one hour at room temperature).

### Summary of the Invention

According to the invention, novel bifunctional hydrazine and hydrazide compounds, as well as conjugates thereof, are provided. Methods of labelling the conjugates with metal ions are also provided.

Broadly, the hydrazine and hydrazide compounds can be described as bifunctional aromatic hydrazines or hydrazides having a protein reactive substituent and a negative counterion. A modification of this invention is also provided in which the hydrazine or hydrazide function is protected as a lower alkyl hydrazone.

In another embodiment of the invention, conjugates are formed by reacting bifunctional hydrazine or hydrazide compounds of the invention with macromolecules such as proteins, polypeptides or glycoproteins. The bifunctional compounds react with nucleophilic groups on the macromolecules (e.g. lysine residues) to yield conjugates containing free hydrazine/hydrazide groups.

In a third embodiment, labelled macromolecules comprised of conjugates and metal ions are formed.

In a fourth embodiment, a method is provided for labelling macromolecules by reacting a conjugate of the invention with a metal species.

### Detailed Description of the Invention

The novel hydrazine and hydrazide compounds of the present invention are represented by one of the following formulas (I) or (II): 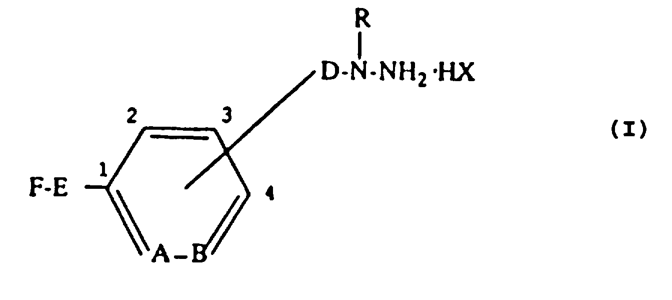 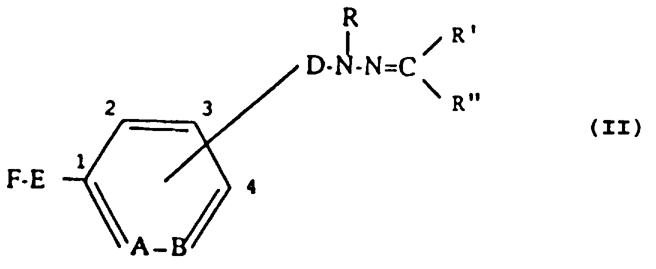 wherein:
A is a carbon or nitrogen atom;
B is a carbon or nitrogen atom:
D is a direct bond (to the 2-, 3-, or 4-position of the ring), CH₂, C=O or 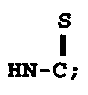
E is C=O or together with F forms a maleimidyl group;
F is selected from the group consisting of N-oxysuccinimidyl, tetrafluorophenolate, N-oxybenztriazole and imidazolate, or
F together with E forms a maleimidyl group;
R is hydrogen or a lower alkyl group;
R' and R" may be the same or different and are selected from hydrogen and lower alkyl; and
X is a negative counterion.

Another embodiment of the invention includes compounds of the formula III: 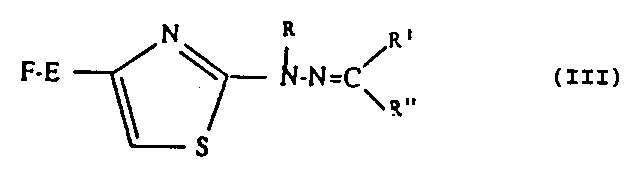 where R, R', R", E and F have the values given above.

Suitable groups for R, R′ and R˝ include, but are not limited to, the following: H, CH₃, C₂H₅, and C₃H₇.

Examples of useful X ions are halides, nitrate, trifluoroacetate, tetrafluoroborate and sulfate. These examples are not intended to limit the scope of suitable counterions.

The above-described compounds are stable, isolable derivatives of molecules that contain two cross-reactive moieties: a hydrazine/hydrazide group and a protein reactive group such as an active ester, active amide or maleimido group.

In the synthesis of these stable derivatives, an acid labile protecting group such as t-butoxycarbonyl (t-BOC) is removed from the hydrazine/hydrazide under anhydrous acidic conditions, leaving the protein reactive group unchanged and the hydrazine/hydrazide group in an unreactive, protonated form. Alternatively, the hydrazine/hydrazide group can be protected as a lower alkyl hydrazone.

When a bifunctional compound having a protonated (or hydrazone protected) hydrazine/hydrazide function is then combined with a macromolecule such as a protein, polypeptide or glycoprotein in neutral or slightly basic media, preferably a pH of about 7-8.5, the protein-reactive part of the compound will react with nucleophilic groups on the protein, polypeptide or glycoprotein (e.g., amine groups such as lysine residues) to yield a conjugate containing free hydrazine/hydrazide groups. In the case of hydrazone conjugates, the free hydrazine/hydrazide is formed by dialysis into an acidic (pH 5.6) buffer. Because this type of conjugate includes a hydrazine or hydrazide, a strong metal binding group, it will then readily react when mixed with a suitable metal species in acidic media to yield a labelled protein, polypeptide or glycoprotein.

The metal species may be, for example, a reduced Tc species formed by reacting TcO₄ ⁻ with a reducing agent, for example, stannous ion, in the presence of a chelating oxygen ligand (e.g. glucoheptonate). Examples of suitable reduced Tc species include Tc-glucoheptonate, Tc-gluconate, Tc-2-hydroxyisobutyrate, Tc-lactate and Tc-4,5-dihydroxy 1,3-benzene disulfonate. Other metals and ligands are also within the scope of the invention.

A Tc labelling process can be conveniently performed in an aqueous buffer, preferably at a pH of about 4.5-6.5, in one hour or less. Reaction with other suitable metal species occurs in a similar manner under similar conditions.

Radiochemical yield as determined by high performance liquid chromatography (HPLC) and thin layer chromatography (TLC) using Tc is ≥ 90%. Treatment of protein with nonlinkable analogs, (i.e., compounds without a protein reactive carbonyl group, such as 4-hydrazinobenzoic acid or 6-hydrazinopyridine-3-carboxylic acid) does not yield protein capable of significant Tc binding, thus demonstrating the high specificity of this technique.

The technetium atoms are believed to be bound to the conjugate via a hydrazide or diazenido linkages:

Protein-linker - N₂TcL₂

or 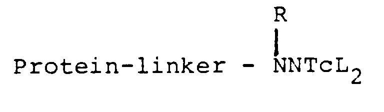 wherein:
L is an ancillary dioxygen ligand.
Examples of this type of linkage have been described for Mo and Re (Comprehensive Coordination Chemistry, Vol. 2, G. Wilkinson ed., Pergamon (Oxford) 1987) p. 130-151 and several analogous complexes of ⁹⁹Tc have been prepared by the reaction of an organohydrazine derivative and a Tc(V) oxo species.

The above labelling scheme has been used to label polyclonal human IgG and the Fc region of human IgG. The Tc-conjugates have been used to image focal sites of infection in a rat model. The labelling scheme has also been used to label fragment E, (see L.C. Knight et al, J. Clin. Invest., Vol. 72 1983, p. 2007-2013 which was used to image thrombi in a rabbit model for deep vein thrombosis and the monoclonal antibody 5E8.

### EXAMPLES

The NMR and IR data given in the examples was obtained as follows:
¹H NMRδ spectra were recorded on an 80 MHz fBM AF-80 Spectrometer. All ¹H NMRδ results were recorded in DMSO-d₆ unless otherwise indicated. Ir spectra were recorded on a Perkin-Elmer 598 infrared spectrometer. NMR and IR spectra were consistent with assigned structure.
Compound names given in brackets below the title compounds in the various examples conform to Chemical Abstracts service index nomenclature. Reaction schemes are illustrated in the accompanying Schemes 1-8.

### EXAMPLE 1:

### Preparation of succinimidyl 4-hydrazinobenzoate hydrochloride [2,5-pyrrolidinedione,1-[(4-hydrazinobenzoyl) oxy]-monohydrochloride] hydrochloride (SHBH).

4-Hydrazinobenzoic acid, 2-(t-butoxycarbonyloxyimino)-2-phenylacetonitrile (BOC-ON), dicyclohexylcarbodiimide and N-hydroxysuccinimide were purchased from Aldrich Chemicals (Milwaukee, WI).

### Synthesis of 4-BOC-hydrazinobenzoic acid

To a stirred solution of 4-hydrazinobenzoic acid (1 equivalent) and triethylamine (3 equivalents) in dimethylformamide (5 mg/l) was added dropwise a solution of BOC-ON (1 equivalent) in dimethylformamide. The reaction mixture was stirred at room temperature for 3 hours. Ten percent aqueous hydrochloric acid was added and subsequently the solution became cloudy. The solution was extracted with ethyl acetate and the combined organic extracts were washed with water, dried over magnesium sulfate, filtered and concentrated under reduced pressure to give a brown solid. The solid was recrystallized from chloroform to give the desired product as a pale brown solid; yield 69%.

| | | | |
|---|---|---|---|
| Analysis: | | | |
| Calculated for C₁₂H₁₆N₂O₄: | C - 57.13; | H - 6.39; | N - 11.10. |
| Found: | C - 57.02; | H - 6.13; | N - 11.61. |

¹H NMR δ: 1.45(s,9H),6.77(d,J_{ab}=8.6Hz,2H), 7.85(d,J_{ab}=8.6Hz,2H)

### Synthesis of succinimidyl 4-BOC-hydrazinobenzoate - [Hydrazinecarboxylic acid. 2-[4-[[(2.5-dioxo-1-pyrrolidinyl) oxylcarbonyl] phenyl]-1,1-dimethylethyl ester]

To a solution of 4-BOC-hydrazinobenzoic acid (1 equivalent) and N-hydroxysuccinimide (1 equivalent) in dioxane (10 ml/g of acid) was added dropwise a solution of dicyclohexylcarbodiimide (1 equivalent) in dioxane (5 ml/g). The reaction mixture was stirred at room temperature for 16 hours. Acetic acid (0.5 ml) was then added and stirring was continued for 1 hour. The reaction mixture was filtered to remove the urea byproduct. The filtrate was concentrated under reduced pressure to give a brown solid which was treated with ether and the solids were isolated by filtration to give a pale brown solid; yield 86%.

| | | | |
|---|---|---|---|
| Analysis: | | | |
| Calculated for C₁₆H₁₉N₃O₆: | C - 55.01; | H - 5.48; | N - 12.03. |
| Found: | C - 55.17; | H - 5.84; | N - 11.86. |

¹H NMR δ: 1.47(s,9H),2.88(s,4H),6.85(d,J_{ab}=8.9Hz,2H) 8.04(d,J_{ab}=8.9Hz,2H)

### Synthesis of succinimidyl 4-hydrazinobenzoate hydrochloride

To a solution of hydrogen chloride in dioxane (50 ml/g of ester; prepared by bubbling hydrogen chloride into dioxane for approximately five minutes) was added succinimidyl 4-BOC-hydrazinobenzoate (1 equivalent). The reaction mixture was stirred at room temperature. The reaction mixture was never homogeneous, however the color was initially pale brown and over 2 hours became orange. The reaction mixture was filtered and washed with ether to give a pale yellow solid; yield 72%; m.p. 203.5 - 205°C.

| | | | | |
|---|---|---|---|---|
| Analysis: | | | | |
| Calculated for C₁₁H₁₂ClN₃O₄: | C - 46.25; | H - 4.23; | C1 - 12.40; | N - 14.71; |
| Found: | C - 46.74; | H - 4.38; | C1 - 12.24; | N - 14.26. |

¹H NMR δ: 2.87(s,4H),7.05(d,2H,J_{ab}=8.9Hz) 7.97 (d,2H,J_{ab}=8.9Hz)

### EXAMPLE 2

### Preparation of succinimidyl 6-hydrazinopyridine-3-carboxylate hydrochloride - [2.5-pyrrolidinedione, 1-[[(6-hydrazino-3-pyridinyl) carbonyl]oxy]-monohydrochloride].

6-chloronicotinic acid, di-t-butyldicarbonate and 85% hydrazine hydrate were purchased from Aldrich Chemicals (Milwaukee, WI).

### Synthesis of 6-hydrazinopyridine-3-carboxylic acid

6-Chloronicotinic acid (8.0g) was added to 85% hydrazine hydrate (35 ml). The reaction mixture was placed in a 100°C oil bath for 4 hours. The homogeneous reaction mixture was concentrated to dryness to give a white solid. The solid was dissolved in water and on acidification to pH 5.5 with concentrated hydrochloric acid a precipitate formed. The precipitate was isolated by filtration and the solid was washed with 95% ethanol and ether to give 6.0 g of a pale brown solid; yield 77% m.p. 292-293°C;

| | | | |
|---|---|---|---|
| Analysis: | | | |
| Calculated for C₆H₇N₃O₂; | C - 47.06; | H - 4.61; | N - 27.44; |
| Found: | C - 46.83; | H - 4.38; | N - 27.27. |

¹H NMR δ: 6.69(d,J=8.8Hz,1H),7.84(dd,J=2.4,8.8Hz,1H), 8.51(d,J=2.4Hz,1H)

### Synthesis of 6-BOC-hydrazinopyridine-3-carboxylic acid

To a solution of 6-hydrazinopyridine-3-carboxylic acid (1.4 g; 9.8 mmol); triethylamine (1.2 ml; 11.8 mmol) in dimethylformamide (10 ml) was added di-t-butyldicarbonate (2.13 g; 9.8 mmol). The reaction mixture became homogeneous over 1 hour and stirring was continued for 16 hours at room temperature. The reaction mixture was concentrated to dryness under reduced pressure to give a brown solid. The residue was dissolved in a minimum amount of ethyl acetate and filtered through silica gel 60 (230-400 mesh) using ethyl acetate as eluent. The eluent was concentrated to dryness. The product was used without further purification.
¹H NMR δ: 1.40(s,9H),6.52(d,J=8.8Hz,1H) 7.97(dd,J-2.4,8.8Hz,1H),8.58(d,J=2.4Hz,1H)

### Synthesis of succinimidyl 6-BOC-hydrazinopyridine-3-carboxylate [Hydrazinecarboxylic acids, 2-[5-[[(2,5-dioxo-1-pyrrolidinyl)oxylcarbonyl]-2-pyridinyl]-, 1,1-dimethylethyl ester]

To a solution of 6-BOC-hydrazinopyridine-3-carboxylic acid (1.45 g; 5.75 mmol), N-hydroxysuccinimide (0.66 g; 5.75 mmol) in dimethylformamide (15 ml) was added a solution of dicyclohexylcarbodiimide (1.18 g; 5.75 mmol) in dimethylformamide (5 ml). The reaction mixture became cloudy after 1 hour and stirring was continued for 16 hours at room temperature. The reaction mixture was filtered and the filtrate was concentrated to dryness to give a brown solid residue. The residue was dissolved in a minimum amount of ethyl acetate and filtered through silica gel 60 (230-400 mesh) using ethyl acetate as eluant. The eluant was concentrated to dryness to give a pale yellow solid which was recrystallized from ethyl acetate/hexanes; yield 60%; m.p. 169.5-172°C;

| | | | |
|---|---|---|---|
| Analysis: | | | |
| Calculated for C₁₅H₁₈N₄O₆; | C - 51.43; | H - 5.18; | N - 15.99 |
| Found: | C - 51.81; | H - 5.26; | N - 15.60. |

¹H NMR δ: 1.41(s,9H),2.87(s,4H)6.64(d,J=8.8Hz,1H) 8.08(dd,J=2.4,8.8Hz)8.73(d,J=2.4Hz,H1)

### Synthesis of succinimidyl 6-hydrazinopyridine-3-carboxylate hydrochloride

A solution of hydrogen chloride in dioxane was prepared by bubbling anhydrous hydrogen chloride into dioxane (20 ml) at a moderate rate for 10 min. Succinimidyl 6-BOC-hydrazinopyridine-3-carboxylate (100 mg) was dissolved in dioxane (2 ml) and HCl/dioxane (2 ml) was added and the reaction mixture was stirred at room temperature. After 5 minutes the solution became cloudy and a precipitate formed. Stirring was continued for 4 hours. The cloudy reaction mixture was filtered to give 55 mg of a white solid; yield 67%;

| | | | | |
|---|---|---|---|---|
| Analysis: | | | | |
| Calculated for C₁₀H₁₁ClN₄O₄; | C - 41.87; | H - 3.87; | Cl - 12.37; | N - 19.53; |
| Found: | C - 41.92; | H - 3.90; | Cl - 12.30; | N - 19.47. |

¹H NMR δ: 2.88(s,4H),7.01(d,J=8.8Hz,1H) 8.19(dd,J=2.4,8.8Hz,1H)8.83(d,J=2.4Hz,1H)

### EXAMPLE 3:

### Preparation of succinimidyl 4-hydrazidoterephthalate hydrochloride - [Benzoic acid, 4-[[(2,5-dioxo-1-pyrrolidinyl) oxy]carbonyl]-, hydrazide, monohydrochloride].

Mono-methyl terephthalate, oxalyl chloride, t-butyl carbazate, dicyclohexylcarbodiimide (DCC) and N-hydroxysuccinimide (NHS) were purchased from Aldrich Chemicals (Milwaukee, WI).

### Synthesis of methyl terephthalate chloride

To a stirred solution of mono-methyl terephthalate (1 equivalent), toluene (30 ml/gm of ester) and 3 drops of DMF was added dropwise oxalyl chloride (2.0 equivalents). The reaction mixture was stirred at 45° C for 16 hours. The solution was concentrated under reduced pressure to give the desired product as a pale yellow solid. The product was used without further purification; yield 82.0%; m.p. 50-52°C. IR (thin film); 2970, 1775, 1720, 1430, 1400, 1280, 1105, 880 cm⁻¹.
¹H NMR δ : 3.97(s,3H),8.14(s,4H).

### Synthesis of methyl 4-BOC-hydrazidoterephthalate - [1,4-benzene-dicarboxylic acid, monomethyl ester, 2-[(1,1-dimethylethoxy) carbonylhydrazidel

To a vigorously stirred mixture of t-butyl carbazate (1 equivalent), methylene chloride (20 ml/gm) and 25% sodium bicarbonate (2.0 equivalents) was added dropwise a solution of methyl terephthalate chloride (1 equivalent) in methylene chloride (40 ml/gm). The reaction mixture was stirred at 20°for 1/2 hour. The phases were separated and the aqueous phase was extracted with methylene chloride. The combined organic phases were washed with 10% hydrochloric acid and brine. The organic phase dried (MgSO₄), filtered and concentrated to give a white solid; yield 91.7%; m.p. 197-199°. IR (KBr): 3010, 1720, 1670, 1430, 1270 1220, 1130, 1100, 1030, 870, 750 cm⁻¹. ¹H NMR δ: (CDCl₃): 1.49 (s,9H), 3.93(s,3H), 7.83(d,J_{ab}=8Hz,2H),8.07(d,J_{ab}=8Hz,2H).

### Synthesis of 4-BOC-hydrazidoterephthalic acid

To a solution of methyl 4-BOC-hydrazidoterephthalate (1 equivalent) in methanol (50 ml/gm) was added sodium hydroxide (10.0 equivalents). The reaction was stirred at room temperature for 16 hours. The reaction mixture was concentrated under reduced pressure to remove the methanol. Water was added and the solution was carefully acidified to pH 1.0. The acidic solution was extracted with ethyl acetate and the organic extract was dried (MgSO₄), filtered and concentrated to dryness under reduced pressure to give a white solid; yield 87.5%; m.p. 208-210°.
¹H NMR δ: 1.41 (s,9H), 7.97 (d,J=2.4Hz,4H), 8.90(m,1H) 10.3(m,1H).

### Synthesis of succinimidyl 4-BOC-hydrazidoterephthalate [Hydrazinecarboxylic acid, 2-[4-[[(2,5-dioxo-1-pyrrolidinyl)oxy] carbonyl] benzoyl]-,1,1-dimethylethyl, ester]

To a solution of 4-BOC-hydrazidoterephthalic (1 equivalent) and N-hydroxy-succinimide (1 equivalent) in DMF (10 ml/gm) was added dropwise a solution of DCC (1 equivalent) in DMF (5 ml/gm). The reaction mixture was stirred at 20° for 16 hours. Acetic acid (0.5 ml) was then added and stirring was continued for 1 hour. The reaction mixture was filtered to remove the urea byproduct. The Filtrate was concentrated under reduced pressure to give a yellow brown oil. Flash vacuum chromatography (hexanes/ethyl acetate (7/3)) was used to isolate the product; yield 47.8%; m.p. 182-185°. IR (KBr): 3330, 3230, 2990, 1770, 1740, 1660, 1530, 1500, 1370, 1280, 1200, 1150, 1070, 1000, 870, 790, 640 cm⁻¹.
¹H NMR δ:(CDCl₃) :1.50(s,9H), 2.91(s,4H), 6.70(m,1H), 7.91 (d,J_{ab}=8.8Hz,2H), 8.20(d,J_{ab}=8.8Hz,2H).

| | | | |
|---|---|---|---|
| Analysis: | | | |
| Calculated for C₁₇H₁₉N₃O₇: | C - 54.11; | H - 5.07; | N - 11.13; |
| Found: | C - 53.66; | H - 5.15; | N - 11.09. |

### Synthesis of succinimidyl-4-hydrazidoterephthalate hydrochloride (SHTH)

To a solution of hydrogen chloride in tetrahydrofuran (50 ml/gm; prepared by bubbling hydrogen chloride into tetrahydrofuran for approximately ten minutes) was added succinimidyl 4-BOC-hydrazidoterephthalate (1 equivalent). The reaction mixture was homogeneous for 1 hour; then over 4 hours a pale white precipitate formed. The reaction mixture was filtered and washed with ether to give the desired product as a pale white solid; yield 37.7%; m.p. 278-280°. IR(KBr): 3400, 3200, 2800, 2600, 1770, 1730, 1690, 1530, 1490, 1290, 1240, 1070, 1000, 870, 730, 640, 610 cm⁻¹. ¹H NMR δ: 2.91(s,4H),8.11(d,J_{ab}=8.8Hz,2H), 8.25(d,J_{ab}=8.8Hz,2H).

| | | | | |
|---|---|---|---|---|
| Analysis: | | | | |
| Calculated for C₁₂H₁₂C1N₃O₅: | C - 45.95; | H - 3.86; | Cl - 11.30; | N - 13.40; |
| Found: | C - 45.84; | H - 3.91; | Cl - 11.37; | N - 13.33. |

### EXAMPLE 4

### Preparation of 5-maleimidyl-2-hydrazinopyridine hydrochloride [1H-pyrrole-2,5-dione, 1-(6-hydrazino-3-pyridinyl)-,monohydrochloride].

2-chloro-5-nitropyridine, hydrazine hydrate, di-tert-butyl dicarbonate, 10% palladium on charcoal, maleic anhydride, cobalt acetate and acetic anhydride were purchased from Aldrich Chemicals (Milwaukee, WI).

### Synthesis of 2-hydrazino-5-nitropyridine

To a stirred solution of hydrazine hydrate (30.0 equivalents), water (4 ml/gm of pyridine), and ethanol (2 ml/gm of pyridine) was added 2-chloro-5-nitropyridine (1 equivalent). The reaction mixture was stirred at 20° for 16 hours (a very thick green slurry forms). The precipitate was isolated by filtration and the solid was washed with methanol and then ether to give a green solid. The product was used without further purification; yield 77.3%; m.p. 205-207°. IR (KBr): 3340, 3200, 2980, 1670, 1605, 1580, 1485, 1420, 1330, 1300, 1120, 980, 830, 77 cm⁻¹.
¹H NMR δ: 4.64(brs,2H),6.76(d,J_{ab}=8.8Hz,1H), 8.15(dd,J=2.4,8.8Hz,1H), 8.86(d,J_{ab}=2.4Hz,1H), 9.12, (m,1H).

| | | | |
|---|---|---|---|
| Analysis: | | | |
| Calculated for C₅H₆N₄O₂: | C - 39.21; | H - 3.93; | N - 36.51; |
| Found: | C - 38.96; | H - 3.92; | N - 36.15. |

### Synthesis of 2-[BOC hydrazino]-5-nitropyridine

To a stirred solution of 2-hydrazino-5-nitropyridine (1 equivalent), DMF (15 ml/gm of pyridine), and triethylamine (1.1 equivalents) was added dropwise a solution of di-tert-butyl dicarbonate (1.0 equivalent) in DMF (4m l/gm of dicarbonate). The reaction mixture was stirred at 20° for 48 hours. The reaction mixture was concentrated under reduced pressure to a yellow brown oil. Flash vacuum chromatography (hexanes/ethyl acetate (8/2)) was used to isolate the product. The product was recrystallized from ethyl acetate/hexanes; yield 63.4%; m.p. 135-137°. IR (KBr): 3280, 2980, 1710, 1600, 1500, 1330, 1290, 1270, 1250, 1150, 1120, 1010, 830, 760, 650, 500 cm₋₁.
¹H NMR δ: 1.41(s,9H),6.60(d,J_{ab}=8.8Hz,1H), 8.28(dd,J=2.4,8.8Hz,1H),8.93(d,J,b=2.4Hz,1H), 9.14(m,1H),9.56(m,1H).

| | | | |
|---|---|---|---|
| Analysis: | | | |
| Calculated for C₁₀H₁₄N₄O₄: | C - 47.24; | H - 5.55; | N - 22.03; |
| Found: | C - 46.99; | H - 5.50; | N - 21.93. |

### Synthesis of 2-(BOC hydrazino)-5-amino-pyridine

Into a Parr hydrogenation bottle was added 2-BOC-hydrazino-5-nitropyridine (1 equivalent), 10% palladium on charcoal (0.3 gm Pd/gm of pyridine) and ethanol (100 ml/gm of pyridine). The reaction was hydrogenated at 50 psi for 2 hours at room temperature on a Parr hydrogenator. The reaction mixture was filtered through a filter cell plug and rinsed with ethanol. The yellow green solution was concentrated under reduced pressure to give a pale yellow solid. The product was recrystallized from ethanol; yield 81.64%; m.p. 140-142°. IR (KBr) 3360, 3200, 2980, 1650, 1635, 1580, 1490, 1390, 1360, 1300, 1260, 1160, 1020, 880, 860, 830, 750, 590, 530 cm⁻¹.
¹H NMR δ: 1.37(s,9H),6.34(d,J_{ab}=8.8Hz,1H), 6.89(dd,J=2.4,8.8Hz,1H),7.14(m,1H), 7.49(d,J_{ab}=2.4Hz,1H),8.50(m,1H).

| | | | |
|---|---|---|---|
| Analysis: | | | |
| Calculated for C₁₀H₁₆N₄O₂: | C - 53.55; | H - 7.19; | N - 24.98; |
| Found: | C - 53.73; | H - 7.21; | N - 25.05. |

### Synthesis of 2-BOC-hydrazino-5-maleimidylpyridine [Hydrazine-carboxylic acid, 2-[5-(2,5-dihydro-2,5-dioxo-1H-pyrrol-1-yl)-2-pyridinyl] -1,1-dimethylethyl ester]

To a stirred solution of 2-BOC-hydrazino-5-amino pyridine (1 equivalent) in acetone (50 ml/gm) was added maleic anhydride (1.1 equivalents). The reaction mixture was stirred at 25° for 2 hours. To the reaction mixture was added acetic anhydride (1.2 equivalents), cobalt acetate (0.007 equivalents) and triethylamine (0.3 equivalents). The reaction mixture was stirred at 60° for 2 hours. The color of the reaction began as bright yellow and ended as dark yellow. The reaction mixture was concentrated under reduced pressure to give a yellow brown oil. Flash vacuum chromatography (hexanes/ethyl acetate (8/2)) was used to isolate the product. The product was recrystallized in ether/hexane; yield 28.3%; m.p. 182-184°. IR (KBr): 3400, 3300, 3100, 2990, 1700, 1610, 1500, 1410, 1360, 1320, 1270, 1210, 1150, 1050, 830, 750, 690 cm⁻¹.
¹H NMR δ: 1.39(s,9H),6.58(d,J_{ab}=8.8Hz,1H), 7.14(s,2H)7.45(dd,J=2.4,8.8Hz,1H), 7.94(d,J_{ab}=2.4Hz,1H),8.37(m,1H).

| | | | |
|---|---|---|---|
| Analysis: | | | |
| Calculated for C₁₄H₁₆N₄O₄: | C - 55.26; | H - 5.30; | N - 18.41; |
| Found: | C - 55.14; | H - 5.30; | N - 18.33. |

### Synthesis of 5-maleimidyl-2-hydrazinopyridine hydrochloride

A solution of hydrogen chloride in dioxane was prepared by bubbling anhydrous hydrogen chloride into dioxane (50 ml) at a moderate rate for 10 minutes. 2-BOC-hydrazino-5-maleimidylpyridine (200 mg) was dissolved in dioxane (5 ml) and HYCl/dioxane (10 ml) was added and the reaction mixture was stirred at room temperature. After 30 minutes the solution became cloudy and a precipitate formed. The reaction mixture was stirred at 25° for a total of 5 hours. The slurry was filtered and washed with ether to give 50 mg of a white solid; yield 31.6%; m.p. 280-290° (decomp.; yellow to brown). IR (KBr): 3440, 3100, 2580, 1720, 1610, 1560, 1480, 1390, 1200, 1150, 830, 690 cm⁻¹.
¹H NMR δ: 7.0(d,J_{ab}=8.8Hz,1H),7.19(s,2H), 7.63(dd,J=2.4,8.8Hz,1H),8.15(d,J_{ab}=2.4Hz,1H).
Mass spectrum: m/z = 204 (M-HC1)+.

### EXAMPLE 5

### Preparation of Succinimidyl 2-(2-propenylhydrazone) nicotinate [Propoanal, [5-[[(2,5-dioxo-1-pyrrolidinyl) oxy]carbonyl]-2-pyridinyl]hydrazone].

6-Hydrazinonicotinic acid was prepared as previously described, and propionaldehyde was purchased from Aldrich Chemicals (Milwaukee, WI).

### Synthesis of Succinimidyl 2-(2-propenylhydrazone) nicotinate -

To a suspension of 6-hydrazinonicotinic acid (1 equivalent) in DMF (40 ml/g) was added propionaldehyde (3 equivalents). The reaction mixture was stirred at ambient temperature for 1 hour. If the reaction mixture did not become homogeneous the flask was gently heated with a heat gun until the reaction mixture became homogeneous. The reaction mixture was cooled to ambient temperature and a solution of N-hydroxysuccinimide (1 equivalent) in DMF was added. Subsequently a solution of DCC (1 equivalent) in DMF was added dropwise via a pressure-equalizing addition funnel. The reaction mixture was stirred for 16 hours at ambient temperature. The precipitate which formed was removed by suction filtration and the mother liquors were concentrated to dryness. The brown solid residue was suspended in ethyl acetate and stirred for 1 hour and filtered. A pale brown solid precipitated from the ethyl acetate solution and was isolated by filtration to give the desired product; yield 65%.
¹H NMR δ: 1.06(t,3H),2.34(m,2H),2.86(s,4H), 7.11(d,J_{ab}=9.4Hz,1H),7.54(t,1H,J=4.9Hz), 8.10(dd,J=9.44,2.33Hz,1H),8.72(d,J=2.33Hz,1H).

| | | | |
|---|---|---|---|
| Analysis: | | | |
| Calculated for C₁₃H₁₄N₄O₄: | C - 53.79; | H - 4.86; | N - 19.30; |
| Found: | C - 53.66; | H - 4.89; | N - 19.12. |

### EXAMPLE 6

### Preparation of Succinimidyl 2-(2-(1-propenyl)hydrazone))-thiazole-4-carboxylate [Propanal, [4-[[(2,5-dioxo-1- pyrrolidinyl)oxylcarbonyl]-2-thiazolyl]hydrazone]

Thiosemicarbazide and bromolactic acid were purchased from Aldrich Chemicals (Milwaukee, WI).

### Synthesis of Propionaldehyde Thiosemicarbazone

To a solution of thiosemicarbazide (1 equivalent) and propionaldehyde (1.5 equivalents) in MeOH was added a few drops of glacial acetic acid. The mixture was heated to reflux for 45 minutes. The reaction mixture was concentrated on the rotavap which caused a precipitate to form. The solids were isolated by filtration, washed with ether and dried in vacuo; yield 71%.

### Synthesis of 2-(2-(1-propenylhydrazone)) thiazole-5-carboxylic acid hydrobromide

To a solution of propionaldehyde thiosemicarbazone (1 equivalent in MeOH was added bromolactic acid (1 equivalent). The reaction mixture was heated at reflux for 1 hour, then cooled to room temperature and the solvent was removed on the rotavap. The resulting yellow solid was triturated with MeOH/ether and a pale yellow solid was isolated and washed with ether and dried in vacuo.
¹H NMR δ: 1.00(t,J=7.9Hz,3H),2.15(m,2H), 7.44(t,J=4.9Hz).

### Synthesis of Succinimidyl 2-(2-(1-propenyl) hydrazone)-thiazole-5-carboxylate

To a solution of acid (1 equivalent) and triethylamine (1.5 equivalents) in DMF was added dropwise a solution of DCC (1 equivalent) in DMF. The reaction mixture was stirred for 16 hours at room temperature. The precipitate which formed was removed by filtration and the mother liquors were concentrated to dryness. Ethyl acetate was added to the residue and stirred for 1 hour. The insolubles were removed by filtration and the mother liquor was concentrated to dryness. The product was flash chromatographed using hexanes/ethyl acetate (1/2) as eluant to give the desired product as an off-white solid; yield 35%.
¹H NMR δ: 1.08(t,J=7.9Hz,3H),2.24(m,2H), 7.40(t,J=4.9Hz,1H),8.11(s,1H).

### EXAMPLE 7:

### Preparation of succinimidyl 2-(2-methylethenylhydrazone) thiazole-4-carboxylate

### Synthesis of succinimidyl 2-(2-methylethenylhydrazone) thiazole-4-carboxylate

-2-(methylethyenylhydrazone)-5-thiazolecarboxylic acid hydrobromide (1 equivalent) (prepared according to the method of H Johne, D. Seifert, S. Johne and E. Bulka, Pharmazie 33, 259 (1978) was dissolved in DMF (20 ml/g). N-hydroxysuccinimide (1 equivalent) and triethylamine (1.5 equivalents) were added. To the homogeneous mixture a solution of DCC (1 equivalent) was added dropwise over 15 minutes. The reaction mixture was stirred for 16 hours at room temperature. The precipitate which formed was removed by filtration and the mother liquor was concentrated to dryness to give an orange-brown solid. The residue was suspended in ethyl acetate and stirred at room temperature for 1 hour. Insolubles were removed by filtration and the mother liquor was concentrated to give a brown solid. The solids were triturated with ether and reisolated by filtration to give a yellow-brown solid; yield 40%. A sample of the product was filtered through a short plug of silica gel using hexanes/ethyl acetate (2/1) as eluate. The eluant was concentrated to give the desired product as a pale yellow solid; m.p. 202-205° (decomp.).
¹H NMR δ: 1.92(s,3H),1.96(s,3H),2.86(s,4H), 8.11(s,1H).
Mass spectrum: m/z = 297 (M+1)+

### EXAMPLE 8:

### Preparation of Succinimidyl 4-thiosemicarbazidobenzoate hemihydrochloride [Hydrazinecarbothioamide, N-[4-[[(2,5-dioxo-1-pyrrolidinyl)oxyl]carbonyl]-phenyl]-, hemihydrochloride].

4-Aminocarboxylic acid was purchased from Aldrich Chemicals (Milwaukee, WI).

### Synthesis of BOC-4-thiosemicarbazidobenzoic acid. [Hydrazine-carboxylic acid, 2-[[(4-carboxyphenyl) amino]thioxomethyl]-, 1-(1,1-dimethylethyl) ester]

To a solution of 4-isothiocyanatobenzoic acid (1 equivalent) (prepared according to the method of D.W. Browne and G.M. Dyson, J.Chem.Soc. 178 (1934) and triethylamine (1.2 equivalents) in DMF was added to a solution of t-butyl carbazate (1 equivalent). The reaction mixture was stirred at room temperature for 3 hours and subsequently concentrated to dryness. The residue was dissolved in ethyl acetate and washed with 10% citric acid and brine. The organic phase was dried (MgSO₄), filtered and concentrated to give the desired product as an off-white solid; yield 70%; m.p. 131-133° (decomp). ¹H NMR δ: 1.42(s,9H),7.67(d,J_{ab}=8.9Hz,2H), 7.87(d,J_{ab}=8.9Hz,2H).

### Synthesis of Succinimidyl BOC-4-thiosemicarbazidobenzoate [Hydrazinecarboxylic acid, 2-[[[4-[[(2,5-dioxo-1-pyrrolidinyloxyl]carbonyl] phenyl]amino]thioxomethyl]-1,1-dimethylethyl ester]

To a solution of acid (1 equivalent) and N-methylmorpholine (1.1 equivalents) in acetonitrile was added a solution of N-succinimidyl tetrachlorethyl carbonate (1 equivalent) in acetonitrile. The reaction mixture was stirred at room temperature for 16 hours. Ethyl acetate was added to the reaction mixture and the homogeneous solution was washed with cold 5% citric acid, cold water, cold aqueous saturated sodium bicarbonate solution, cold water and cold brine. The organic phase was dried (MgSO₄), filtered and concentrated to give a pale yellow oil. The oil was flash chromatographed on silica gel using hexanes/ethyl acetate as eluant. The product was isolated as a yellow oil which solidified on the addition of ether. The solids were isolated by filtration to give the desired product; yield 25%; m.p. 161-163°.
¹H NMR δ: 1.52(s,9H),2.88((s,4H) 7.74(d,J_{ab}=10.0Hz,2H),8.05(d,J_{ab}10.0Hz,2H).

| | | | | |
|---|---|---|---|---|
| Analysis: | | | | |
| Calculated for C₁₇H₂₀N₄O₆: | C - 49.99; | H - 4.94; | N - 13.72; | S - 7.85; |
| Found: | C - 50.05; | H - 4.95; | N - 13.64 | S - 7.95. |

### Synthesis of Succinimidyl 4-thiosemicarbazidobenzoate hemihydrochloride

To a suspension of BOC succinimidyl ester in ether was added a solution of dry HC1_{g} in ether (prepared by bubbling HC₁ gas into dry ether). The suspension was stirred at room temperature for 16 hours. The reaction mixture was heterogeneous over the entire course of the reaction. The solids were isolated by filtration to give the desired hydrochloride salt product; yield 80% m.p. 155-160°.
¹H NMR δ: 2.88(s,4H),8.01(s,4H).

| | | | | |
|---|---|---|---|---|
| Analysis: | | | | |
| Calculated for C₁₂H₁₃N₄SO .0.5 HCl: | C - 44.00; | H - 4.15; | N - 17.10 | S - 9.79; |
| Found: | C - 44.56; | H - 3.88; | N - 17.04 | S - 9.74. |

### EXAMPLE 9:

### Conjugation of IgG

To a solution of 10 mg IgG (MW = 155,000) in 2 ml 0.1 M sodium phosphate buffer (pH 7.8) was added 17.2 µl of 30 mM succinimidyl 4-hydrazinobenzoate hydrochloride in dimethylformamide. After stirring for 5 hours at room temperature, the reaction mixture was dialyzed against 0.1 M sodium acetate buffer (pH 5.2). The number of hydrazino groups conjugated onto the protein was measured by the method of T.P. King et al. (**Biochemistry, 25**:5774, 1986). Briefly, the hydrazino-protein conjugate was reacted with 4-nitrobenzaldehyde to convert the hydrazino groups into hydrazones. The number of hydrazones/protein molecule were determined spectrophotometrically using the hydrazone of p-nitrophenylbenzaldehyde and phenylhydrazine (λₘₐₓ = 412, ε = 2.41x10⁴) as a standard. Modification yields of 25-35% were obtained.

### EXAMPLE 10:

### Labelling of Conjugated IgG with ^{99m}Tc

A DuPont Tc-glucosan kit was reconstituted with 3 ml of water containing 10mCi of ^{99m}TcO₄ ⁻. 250 µl of this solution was mixed with 250 µl of 1-5 mg/ml conjugated IgG in 0.1M sodium acetate buffer (pH 5.2). After incubation for 1 hour at room temperature > 95% of the activity was associated with the protein as determined by radiometric HPLC (TSK 3000 column) and instant thin layer chromatography (ITLC.)

800 uCi of Tc labelled IgG was injected into rats containing an abscess in one hind leg. At 24 hours the rats were sacrificed. The distribution of radioactivity was measured:

| Organ | % Injected dose/gram tissue |
|---|---|
| Blood | 1.36 |
| Kidney | 1.11 |
| Infected Muscle | 0.76 |
| Normal Muscle | 0.12 |
| Liver | 0.81 |
| Ratio Infected/Normal muscle = 6.3 | |

### EXAMPLE 11

### Conjugation of fragment E₁.

(DD)E protein was concentrated to between 5-10 mg/ml and modified with a 20-fold molar excess of succinimidyl 6-hydrazinopyridine-3-carboxylate hydrochloride in 12.5 mM borate buffer at pH 8.5. After a 5 hour incubation (with gentle stirring) at 4° C, the sample was dialyzed approximately 12 hours versus degassed nanopure water.

Fragment E, was separated from the modified DD(E) complex by making the sample 0.55 M in acetic acid and diluting 1:1 V/V with 6 M urea. The pH of the sample was adjusted to 5.5 with 10 N NaOH and the sample was then dialyzed against 10 mM citrate buffer (pH 5.7) to remove excess reagents. During dialysis, DD protein precipitates leaving modified fragment E, in solution. The DD precipitate was readily removed by centrifugation.

Modified E₁ was labelled with Tc99m via reaction with Tc-99m glucoheptonate, as previously described. The Tc-99m labelled E₁ was used to image a thrombus in a rabbit model (see D. Collen et al., J. Clin. Invest. 71, p. 368-376 (1983)).

### EXAMPLE 12:

### Conjugation of monoclonal antibody 5E8 ( see E.A. Chen et al, Cancer Research, 49, p. 3642-3649 (1989)).

5E8 (at a concentration of 5-10 mg/ml) was modified with a 14-fold excess of succinimidyl 6-hydrazinopyridine-3-carboxylate hydrochloride in 12.5 mM borate buffer at pH 8.5 (5 hours at room temperature). The antibody conjugate was dialyzed against a 20 mM citrate buffer (pH 5.2 100 mM in NaCl). After centrifugation to remove a small amount of turbidity the degree of modification (determined spectrophotometrically, as previously described) was found to be 6.5 hydrazine groups per protein molecule. Analysis by ELISA and immunocytoadherence showed no loss in immunoreactivity.

The specific compounds and details of the method described above are exemplary and are not intended to limit the scope of the invention.

This invention also provides a process for the preparation of a compound of the formula: 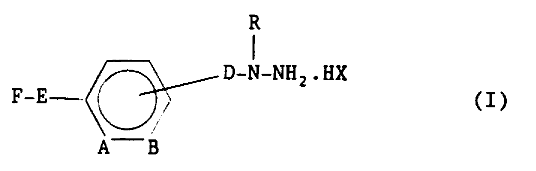 or 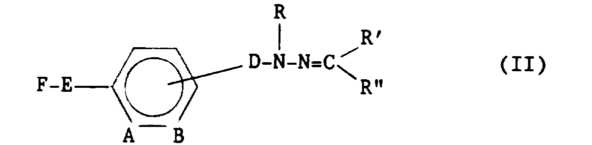 or 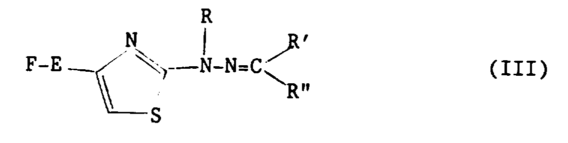 wherein:
A is a carbon or nitrogen atom;
B is a carbon or nitrogen atom;
D is a direct bond (to the 2-, 3-, or 4-position of the ring), CH₂, C=O or 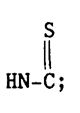
E is a C=O or together with F forms a maleimidyl group;
F is a group readily replaced by a primary amine in neutral or basic aqueous media when E is C=O or together with E
form a maleimidyl group;
R is hydrogen or a lower alkyl group;
R′ and R˝ may be the same or different and are selected from hydrogen and lower alkyl; and
X is a negative counterion,
   which process comprises reacting an optionally protectant reactant of formula 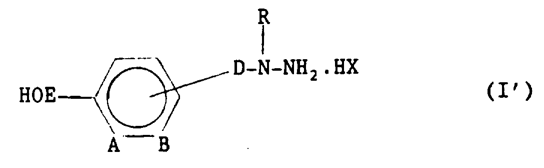 or 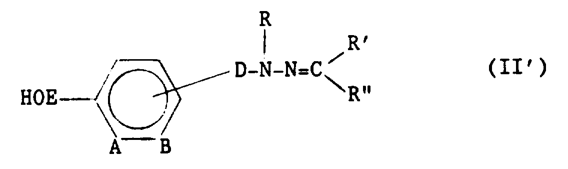 or 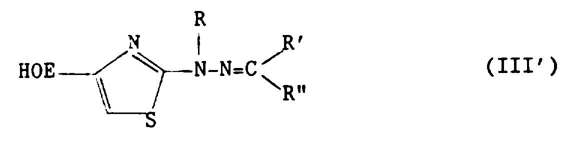 with a chemical of formula FH and removing any protected group; or where F and E together form a maleimidyl group reacting an optionally protected amine of formula 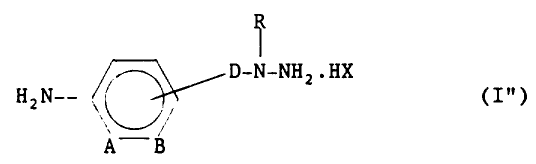 or 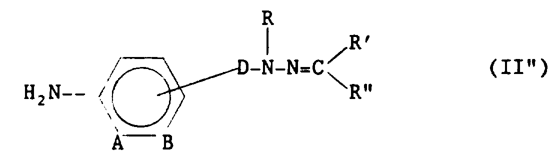 or 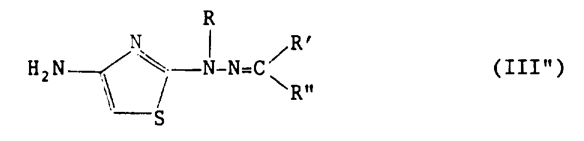 with maleic anhydride and removing any protectant group.

A further process for preparing a conjugate, comprises reacting a macromolecule with a compound of formula I, II or III as defined in above.

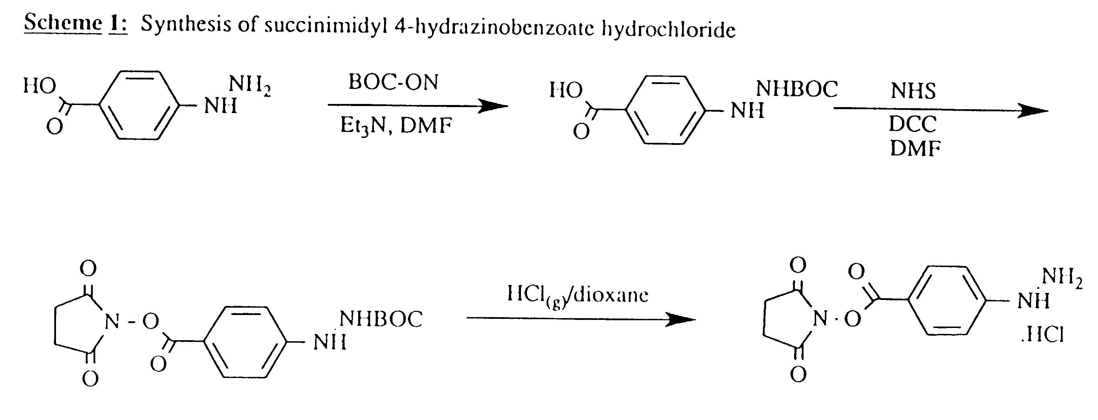

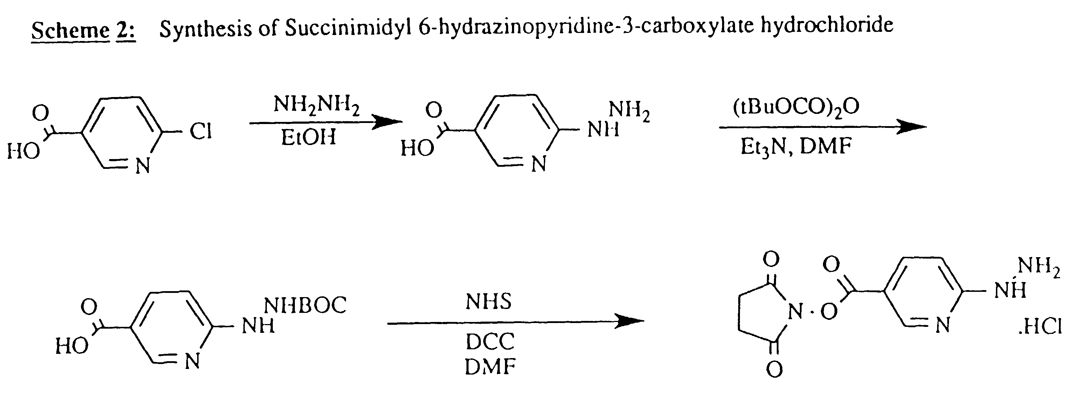

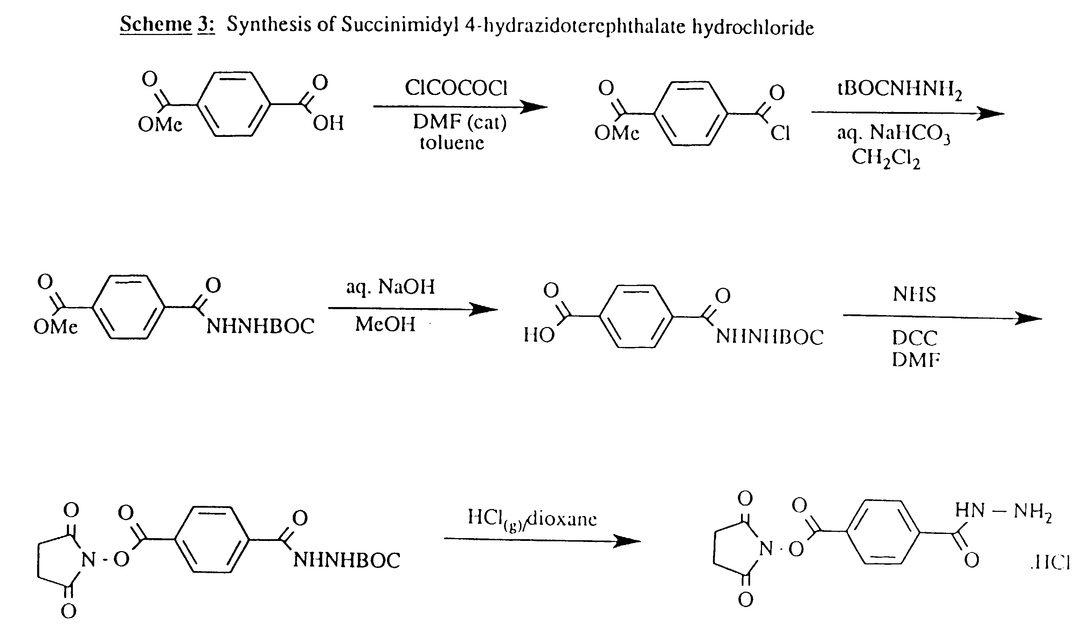

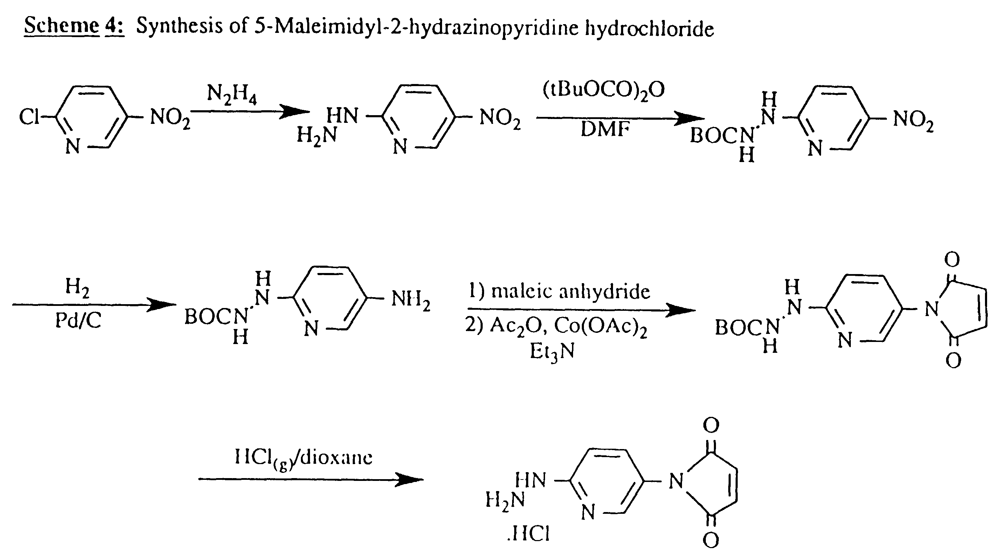

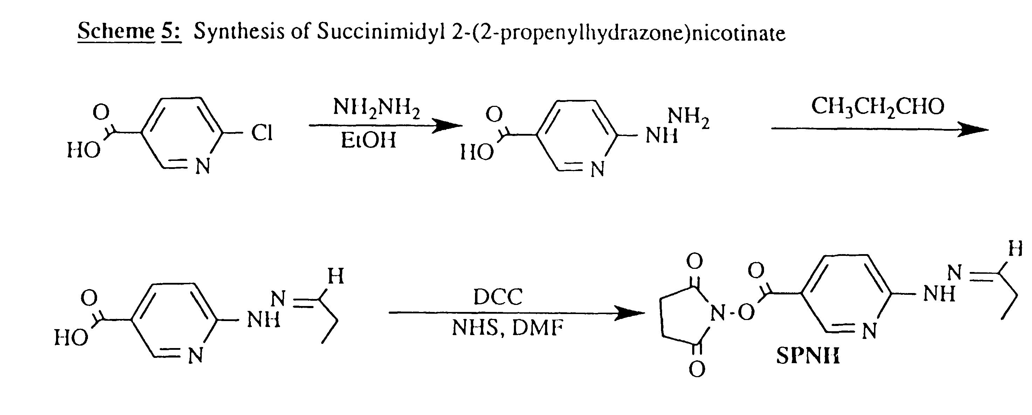

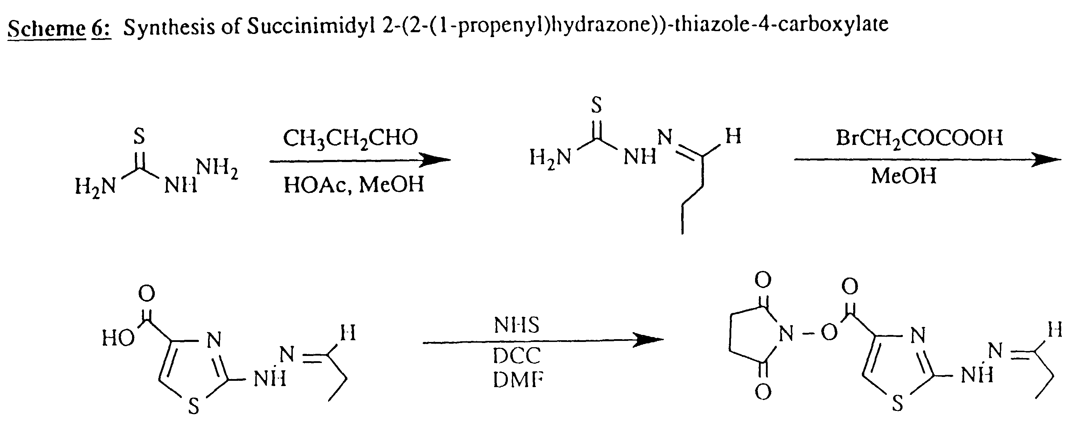

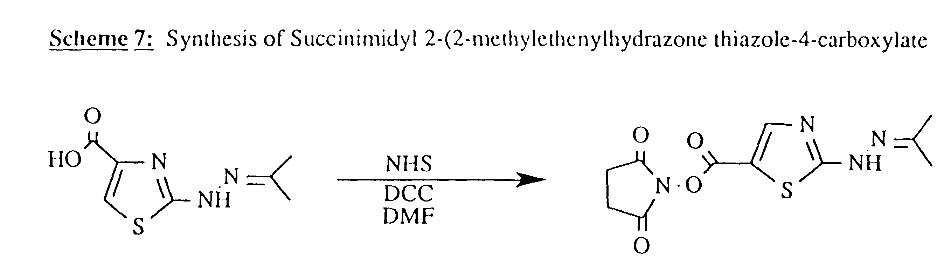

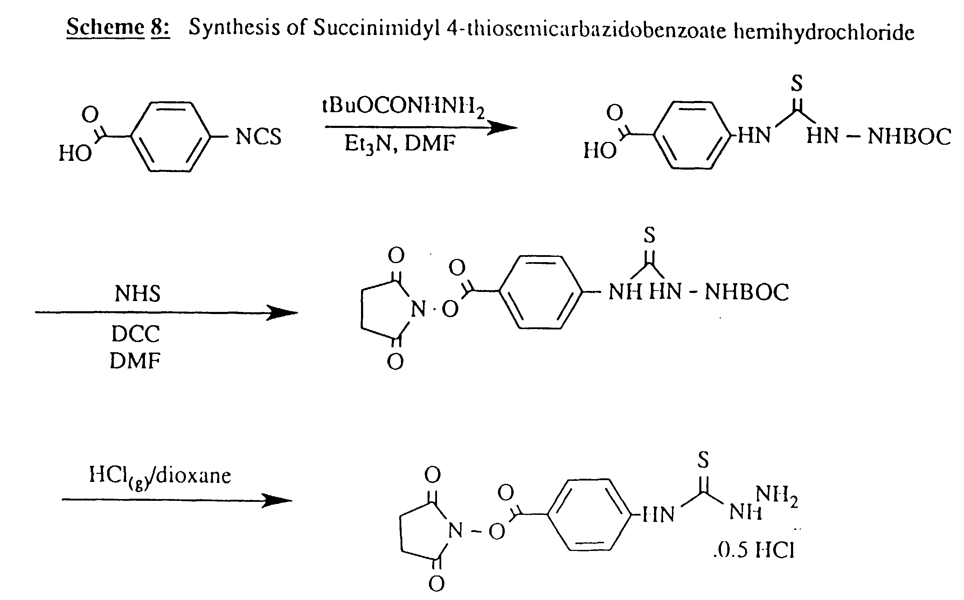

## Claims

1. A hydrazine or hydrazide compound of the formula: 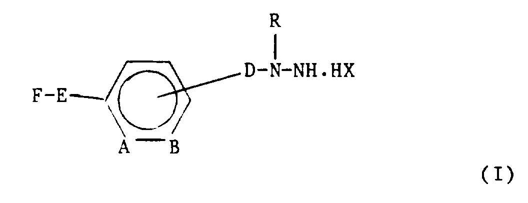 or 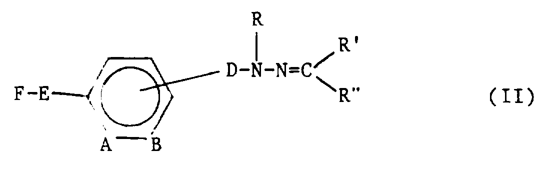 or 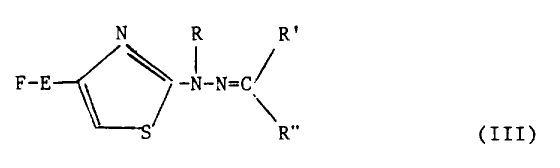 wherein:
A is a carbon or nitrogen atom;
B is a carbon or nitrogen atom:
D is a direct bond (to the 2-, 3-, or 4-position of the ring), CH₂, C=O or 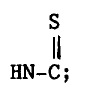
E is C=0 or together with F forms a maleimidyl group;
F is selected from the group consisting of N-oxysuccinimidyl, tetrafluorophenolate, N-oxybenztriazole and imidazolate, or F together with E forms a maleimidyl group;
R is hydrogen or a lower alkyl group;
R' and R" may be the same or different and are selected from hydrogen and lower alkyl; and
X is a negative counterion.

2. A compound according to formula I or II of claim 1 wherein D is a direct bond to the 4-position of the ring.

3. A compound according to claim 2 wherein X is selected from the group consisting of halides, nitrate, trifluoroacetate, tetrafluoroborate and sulfate.

4. A compound according to claim 2, wherein R is hydrogen or methyl, E is carbonyl, F is N-oxysuccinimidyl, and X is Cl.

5. A compound according to claim 4, wherein both A and B are carbon atoms or one of A and B is carbon and the other is nitrogen.

6. A compound according to claim 4, wherein R is hydrogen, and either A is carbon and B is nitrogen or both A and B are nitrogen atoms.

7. A compound according to any of the preceding claims, wherein D is C=O, CH₂ or 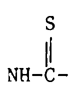 and is attached to the 4-position of the ring.

8. A compound according to claim 7, wherein E is carhonyl, F is N-oxysuccinimidyl, X is Cl, and D is either carbonyl or thioamide.

9. A compound according to claim 2, wherein E is carbonyl, F is N-oxysuccinimidyl, A is carbon, B is nitrogen, R and R' are hydrogen, and R" is ethyl.

10. A compound according to formula III of claim 1, wherein E is carbonyl, F is N-oxysuccinimidyl, and either R' is hydrogen and R" is ethyl, or both R' and R" are methyl.

11. A conjugate formed by reaction of a macromolecule with the hydrazine or hydrazide compound of claim 1.

12. A conjugate according to claim 11, wherein said macromolecule comprises an immunoglobulin or a fragment thereof.

13. A labelled macromolecule comprising a metal ion and a conjugate according to claim 11 or 12.

14. A labelled macromolecule according to claim 13, wherein said metal ion is selected from the group consisting of Tc and Re.

15. A method for labelling macromolecules with metal ions comprising the step of reacting a Tc or Re radioisotope species with a conjugate comprised of at least one macromolecule and a compound according to any one of claim 1 to 10, at conditions sufficient to induce labelling.

16. A method according to claim 15, wherein said metal species comprises a reduced Tc species or a reduced Re species.

17. A method according to claim 16, wherein said reduced Tc species is formed by reacting TcO₄ with a reducing agent in the presence of a chelating oxygen ligand.

18. A method according to claim 17, wherein said chelating oxygen ligand is selected from the group consisting of glucoheptonate, gluconate, 2-hydroxyisobutyrate, lactate and 4,5-dihydroxy-1,3-benzene disulfonate.

19. A method according to claim 17, wherein said reducing agent comprises stannous ion.

20. A method according to any of claims 15 to 19, wherein said at least one macromolecule comprises an immunoglobulin or a fragment thereof and said compound having at least one free hydrazine or hydrazide group is selected from the group consisting of succinimidyl 4-hydrazinobenzoate hydrochloride, succinimidyl 6-hydrazinopyridine-3-carboxylate hydrochloride, succinimidyl 2-(2-propenylhydrazone) nicotinate and succinimidyl 4-thiosemicarbazidoebenzoate hemihydrochloride.

## Patentansprüche

1. Hydrazin- oder Hydrazidverbindung der Formel: 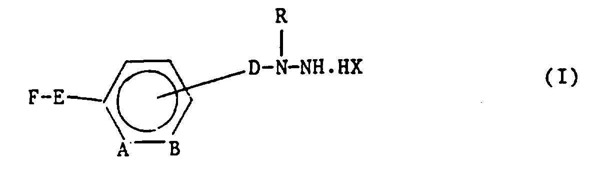 oder 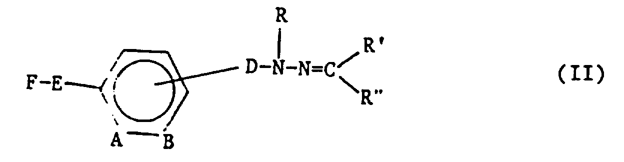 oder 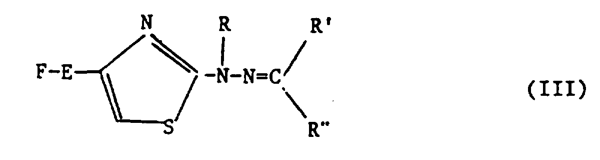 worin
A ein Kohlenstoff- oder Stickstoffatom ist;
B ein Kohlenstoff- oder Stickstoffatom ist;
D eine direkte Bindung (zu der Position 2, 3 oder 4 des Rings), CH₂, C=O oder 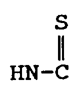 ist;
E C=O ist oder zusammen mit F eine Maleimidylgruppe bildet;
F ausgewählt ist aus der Gruppe bestehend aus N-Oxysuccinimidyl, Tetrafluorphenolat, N-Oxybenztriazol und Imidazolat oder
F zusammen mit E eine Maleimidylgruppe bildet;
R Wasserstoff oder eine Niedrigalkylgruppe ist;
R' und R" gleich oder verschieden sein können und ausgewählt sind aus Wasserstoff und Niedrigalkylgruppen und
X ein negatives Gegenion ist.

2. Verbindung nach Formel I oder II von Anspruch 1, worin D eine direkte Bindung zu Position 4 des Rings ist.

3. Verbindung nach Anspruch 2, worin X ausgewählt ist aus der Gruppe bestehend aus Halogeniden, Nitrat, Trifluoracetat, Tetrafluorborat und Sulfat.

4. Verbindung nach Anspruch 2, worin R Wasserstoff oder ein Methylrest ist, E ein Carbonylrest ist, F ein N-Oxysuccinimidylrest ist und X Cl ist.

5. Verbindung nach Anspruch 4, worin sowohl A als auch B Kohlenstoffatome sind oder einer der Reste A und B Kohlenstoff ist und der andere Stickstoff ist.

6. Verbindung nach Anspruuch 4, worin R Wasserstoff ist und entweder A Kohlenstoff ist und B Stickstoff ist oder sowohl A als auch B Stickstoffatome sind.

7. Verbindung nach einem der vorhergehenden Ansprüche, worin D C=O, CH₂ oder 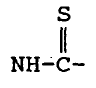 ist und an Position 4 des Rings gebunden ist.

8. Verbindung nach Anspruch 7, worin E ein Carbonylrest ist, F ein N-Oxysuccinimidylrest ist, X Cl ist und D entweder ein Carbonylrest oder ein Thioamidrest ist.

9. Verbindung nach Anspruch 2, worin E ein Carbonylrest ist, F ein N-Oxysuccinimidylrest ist, A Kohlenstoff ist, B Stickstoff ist, R und R' Wasserstoffatome sind und R" ein Ethylrest ist.

10. Verbindung der Formel III nach Anspruch 1, worin E ein Carbonylrest ist, F ein N-Oxysuccinimidylrest ist und entweder R' Wasserstoff ist und R" ein Ethylrest ist oder beide Reste R' und R" Methylreste sind.

11. Konjugat gebildet durch die Reaktion eines Makromoleküls mit der Hydrazin- oder Hydrazidverbindung von Anspruch 1.

12. Konjugat nach Anspruch 11, worin das Makromolekül ein Immunglobulin oder ein Fragment davon umfaßt.

13. Markiertes Makromolekül umfassend ein Metallion und ein Konjugat nach Anspruch 11 oder 12.

14. Markiertes Makromolekül nach Anspruch 13, worin das Metallion ausgewählt ist aus der Gruppe bestehend aus Tc und Re.

15. Verfahren zur Markierung von Makromolekülen mit Metallionen, umfassend die Stufe, daß man ein Molekül mit einem radioaktiven Isotop von Tc oder Re mit einem Konjugat umsetzt, das aus mindestens einem Makromolekül und einer Verbindung nach einem der Ansprüche 1 bis 10 zusammengesetzt ist, unter Bedingungen, die ausreichen, um eine Markierung zu induzieren.

16. Verfahren nach Anspruch 15, worin das Metallmolekül ein reduziertes Tc-Molekül oder ein reduziertes Re-Molekül umfaßt.

17. Verfahren nach Anspruch 16, worin das reduzierte Tc-Molekül gebildet wird, indem TcO₄ mit einem Reduktionsmittel in Gegenwart eines komplexierenden Sauerstoffliganden umgesetzt wird.

18. Verfahren nach Anspruch 17, worin der komplexierende Sauerstoffligand ausgewählt ist aus der Gruppe bestehend aus Glucoheptonat, Gluconat, 2-Hydroxyisobutyrat, Lactat und 4,5-Di-hydroxy-1,3-benzoldisulfonat.

19. Verfahren nach Anspruch 17, worin das Reduktionsmittel ein Zinnion umfaßt.

20. Verfahren nach einem der Ansprüche 15 bis 19, worin mindestens ein Makromolekül ein Immunglobulin oder ein Fragment davon umfaßt und die Verbindung mit mindestens einer freien Hydrazin- oder Hydrazidgruppe ausgewählt ist aus der Gruppe bestehend aus Succinimidyl-4-hydrazinobenzoathydrochlorid, Succinimidyl-6-hydrazinopyridin-3-carboxylathydrochlorid, Succinimidyl-2-(2-propenylhydrazon)-nikotinat und Succinimidyl-4-thiosemicarbazidobenzoathemihydrochlorid.

## Revendications

1. Hydrazine ou hydrazide de formule : 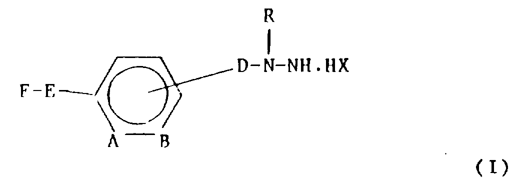 ou 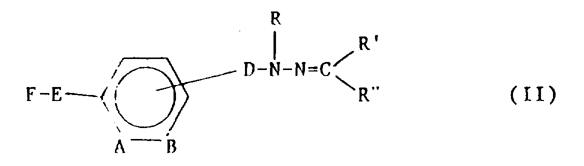 ou 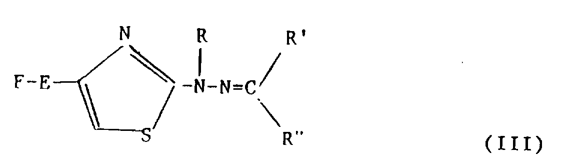 formules dans lesquelles :
A représente un atome de carbone ou d'azote;
B représente un atome de carbone ou d'azote;
D est une liaison directe (sur la position 2-, 3 - ou 4- du noyau) ou représente CH₂, C=O ou 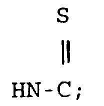
E représente C=O ou, pris avec F, il forme un groupe maléimidyle;
F est choisi dans l'ensemble consistant en un groupe N-oxysuccinimidyle, tétrafluorophénolate, N-oxybenzotriazole et imidazolate, ou bien F forme avec E un groupe maléimidyle;
R représente un atome d'hydrogène ou un groupe alkyle inférieure;
R' et R", qui peuvent être identiques ou différents, sont choisis chacun parmi un atome d'hydrogène et un groupe alkyle inférieur; et
X est un contre-ion négatif.

2. Composé selon la formule I ou la formule II de la revendication 1, dans laquelle D représente une liaison directe fixée sur la position 4 du noyau.

3. Composé selon la revendication 2, dans lequel X est choisi dans l'ensemble consistant en des groupes halogénures, nitrate, trifluoroacétate, tétrafluoroborate et sulfate.

4. Composé selon la revendication 2, dans lequel R représente un atome d'hydrogène ou un groupe méthyle; E représente un groupe carbonyle, F représente un groupe N-oxysuccinimidyle, et X représente Cl.

5. Composé selon la revendication 4, dans le cas duquel A et B représentent chacun un atome de carbone ou bien l'un des A et B représente un atome de carbone et l'autre représente un atome d'azote.

6. Composé selon la revendication 4, dans le cas duquel R représente un atome d'hydrogène et A représente un atome de carbone et B représente un atome d'azote ou bien A et B représentent chacun des atomes d'azote.

7. Composé selon l'une quelconque des revendications précédentes, dans lequel D représente C=O, CH₂ ou 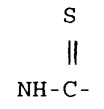 et est fixé sur la position 4 du noyau.

8. Composé selon la revendication 7, dans lequel E représente un groupe carbonyle, F représente un groupe N-oxysuccinimidyle, X représente Cl et D représente un groupe carbonyle ou thioamide.

9. Composé selon la revendication 2, dans lequel E représente un groupe carbonyle, F représente un groupe N-oxysuccinimidyle, A est un atome de carbone, B est un atome d'azote, R et R' représentent chacun un atome d'hydrogène, et R" représente un groupe éthyle.

10. Composé suivant la formule III de la revendication 1, dans laquelle E représente un groupe carbonyle, F représente un groupe N-oxysuccinimidyle, et R' représente un atome d'hydrogène et R" représente un groupe éthyle, ou bien les symboles R' et R" représente chacun un groupe méthyle.

11. Conjugué formé par la réaction d'une macromolécule avec l'hydrazine ou l'hydrazide de la revendication 1.

12. Conjugué selon la revendication 11, dans lequel ladite macromolécule comprend une immunoglobuline ou un fragment d'immunoglobuline.

13. Macromolécule marquée, comprenant un ion de métal et un conjugué selon la revendication 11 ou 12.

14. Macromolécule marquée, selon la revendication 13, dans laquelle ledit ion de métal est choisi dans l'ensemble consistant en Tc et Re.

15. Procédé pour marquer des macromolécules avec des ions de métaux, le procédé comprenant l'étape consistant à faire réagir un radioisotope de Tc ou de Re avec un conjugué formé d'au moins une macromolécule et d'un composé selon l'une quelconque des revendications 1 à 10, dans les conditions suffisantes pour induire du marquage.

16. Procédé selon la revendication 15, dans lequel ladite espèce de métal comprend une espèce de Tc réduit ou une espèce de Re réduit.

17. Procédé selon la revendication 16, dans lequel ladite espèce de Tc réduit est formée par la réaction de TcO₄ avec un agent réducteur, en présence d'un ligand oxygéné chélateur.

18. Procédé selon la revendication 17, dans lequel le ligand oxygéné chélateur est choisi dans l'ensemble consistant en du glucoheptonate, du gluconate, du 2-hydroxyisobutyrate, du lactate et du 4,5-dihydroxy-1,3-benzène disulfonate.

19. Procédé selon la revendication 17, dans lequel ledit agent réducteur comprend, ou est formé par, de l'ion stanneux.

20. Procédé selon l'une quelconque des revendications 15 à 19, dans lequel ladite au moins une macromolécule comprend, ou est constituée par, une immunoglobuline ou un fragment d'immunoglobuline, et ledit composé comportant au moins un groupe hydrazine libre ou hydrazide est choisi dans l'ensemble consistant en un chlorhydrate 4-hydrazinobenzoate de succinimidyle, un chlorhydrate de 6-hydrazinopyridine-3-carboxylate de succinimidyle, du 2-(2-propénylhydrazone) nicotinate de succinimidyle et de l'hémychlorhydrate de 4-thiosemicarbazidobenzoate de succinimidyle.
